Europäisches Patentamt

(19) European Patent Office

Office européen des brevets

(11) Publication number: **0 186 258 B1**

(12) **EUROPEAN PATENT SPECIFICATION**

(45) Date of publication of patent specification: **28.08.91**       (51) Int. Cl.⁵: **A61K 7/48**

(21) Application number: **85303501.2**

(22) Date of filing: **17.05.85**

(54) Lotion for blemished scin (comprising thymol).

(30) Priority: **17.05.84 IL 71864**

(43) Date of publication of application:
**02.07.86 Bulletin 86/27**

(45) Publication of the grant of the patent:
**28.08.91 Bulletin 91/35**

(84) Designated Contracting States:
**CH DE FR GB LI**

(56) References cited:
**DD-A- 123 640**
**US-A- 2 257 106**
**US-A- 2 290 908**

**CHEMICAL ABSTRACTS, vol. 103, 1985, page 320, abstract no. 11238e, Columbus, Ohio, US; & IL-A-71 864 (H. RIVKIN) 30-11-1984**

**CHEMICAL ABSTRACTS, vol. 98, 1983, page 348, abstract no. 40422u, Columbus, Ohio, US; & SU-A-957 907 (L.N. MACHULENKO et al.) 15-09-1982**

**ARTHUR OSOL, Remington's Pharmaceutical Sciences, 16th edition, 1980, page 112, Mack Publishing Company, Easton, Pennsylvania, US**

(73) Proprietor: **Rivkin, Haviva**
**Hapalmach 23/11**
**Holon 58285(IL)**

(72) Inventor: **Rivkin, Haviva**
**Hapalmach 23/11**
**Holon 58285(IL)**

(74) Representative: **Seaborn, George Stephen et al**
**c/o Edward Evans & Co. Chancery House**
**53-64 Chancery Lane**
**London WC2A 1SD(GB)**

## Description

This invention relates to treatment of blemished or scarred skin or inflamed skin with acne (comedones).

The first cosmetic products that come into contact with our skin are of the cleansing and protecting kind. The skin of most children during the early years is healthy, soft, free from spots and blemishes and requires little care other than regular cleansing.

At the onset of puberty, however, the skin becomes susceptible to a range of problems, most of which can be attributed to over-activity of the oil-producing sebaceous glands. It has been proved that more than 50% of those effected by excessive spots, pimples, blackheads, acne and related conditions are adolescents between 12 and 18.

The sebaceous glands together with the muscular, nervous and vascular systems are known as the pilosebaceous apparatus and are largely under the control of endogenous hormones which are present in unusually high concentration in the blood during adolescence and puberty * . The corresponding increase in activity of the sebaceous glands themselves gives rise to the production of excessive amounts of sebum. This causes an oiliness of the skin and an increase in the rate of keratinization of the skin's horny layer (the stratum corneum). The horny cells lining the sebaceous follicles also proliferate; they become tightly-packed and can form an occlusive plug or comedone. This physical barrier, coupled with the increased production of sebum, leads to a rapid accumulation of back pressure and the stagnant sebum forms an ideal medium for the proliferation of bacteria (Staphylococcus albus, Staphylococcus aureus and Corynebacterium acnes). When the plugged follicle ruptures and allows the discharge of its contents, local swelling and inflamation are caused.

So far, no topically active substance has yet been found capable. Acne is usually treated by vitamin A, antibiotics (tetracycline), various hormonal drugs, or subcutaneous hormonal injections.

Thymol has been used for many years as a component of various drugs. Thymol is extracted from the volatile oil of Thymus Vulgaris and is an antiseptic used in vivo and in vitro in suitable dosage, as well as for local anaesthesia.

I have now discovered that thymol and Spiritus vini may be used to prepare a highly effective medicament or cosmetic product for treatment of blemished or scarred skin or skin inflamed with acne (comedones).

In accordance with the present invention, there is provided use of thymol in amount of 0.03-0.75% (by weight) and Spiritus vini in amount of 40-95% (by volume) for preparation of a medicament or cosmetic product for treatment of blemished or scarred skin or skin inflamed with acne (comedones).

The particular efficaciousness of the cosmetic product consists in its independent action upon skin, irrespective of the blemishes' origin (hormonal condition, mood, food quality, condition of the digestive system) and independent of drugs, such as antibiotics, vitamins and hormonal drugs.

Thymol has proved to be a most effective constituent for local external treatment of problem skin (inflamed, scarred or with comedones).

On the basis of experimental practice in problem skin treatment I have reached the conclusion that the thymol component operates as a 'peeling agent' for the rapid and effective removal of keratinized squamous cells of the horny layer whose accumulation foster the formation of comedones.

Treatment by the product of the invention removes the layer of grease from the skin surface and the blemishes disappear in most cases already within one week. The secretion of sebum by the sebaceous glands decreases gradually until it reaches a nearly normal degree.

*Information in this paragraph is from P. Alexander, S.F. Bloomfield et al., eds: Harry's Cosmeticology. (George Godwin: 1982, 7th edition), Chapter Nine.

A'- High degree of sebum secretion by the sebaceous glands

A"- Normal " " " " " " " "

A"' - Low " " " " " " " "

Time - Section of time starting with O (starting point of the experiment), every section corresponding to a week.

During the period of treatment, which in some cases measured up to two years, no cases at all were observed of a reduction of the activity of the glands from a heightened to a low (subnormal) degree.

For a conclusion:

The effect upon problem skin of the medicament or cosmetic product containing thymol and Spiritus vini is as follows:

1. Problem skin (blemished with scars, pimples, comedones, blocked with grease) is freed from all these problems as a result of the treatment, the length of the period of treatment needed varying individually and in proportion to the degree of gravity of the situation. The product entails no addiction; after the treatment is terminated the patients continue using other cosmetic products. (These results are valid for patients aged 18-22).

2. The skin is freed from blemishes with no traces left (such as scars or creases) except for cases where the skin is damaged by other treatment or product.

3. Patients with skin blemished with scars or blotches previous to treatment were relieved from these. Creases disappear completely in all case, whereas the removal of scars or improvement of scarred skin depends on the individual case and is in inverse proportion to the scars long standing previous to treatment with the productof the invention.

4. Treatment with the product of the invention at an early stage (at ages 12-14) prevents acne completely.

5. The product of the invention has the singular advantage of being the only necessary agent during the period of treatment - no supplementary dietary or medical treatment need be involved.

The above mentioned results are true for:

a. a group of patients whose skin problems were never treated before;

b. a group of patients whose case history reveals that conventional medical or cosmetic treatment was of no avail.

According to the invention, the medicament or cosmetic product comprises about 0.03-0.75 g of thymol and 40-95 ml of Spiritus vini per 100 ml of the medicament or cosmetic product. The balance is made up by substances like water and glycerine, which have no effect on either product but constitute the accepted bases for cosmetic liquids or creams.

Cosmetic products according to the invention are prepared by methods known per se in cosmetic technology.

Several examples are given hereunder to illustrate the cosmetic product according to the invention:

EXAMPLE I

## LOTION FOR BLEMISHED SKIN

| Ingredients | Relative Proportions |
| --- | --- |
| Thymol | 0.03 g |
| Spiritus Vini | 95.00 ml |
| Aqua Distillata | 2.97 ml |
| Glycerine | 2.00 ml |
| | 100.00 ml |

EXAMPLE II

## LOTION FOR BLEMISHED SKIN

| Ingredients | Relative Proportions |
| --- | --- |
| Thymol | 0.75 g |
| Spiritus Vini | 40.00 ml |
| Aqua Distillata | 50.25 ml |
| Glycerine | 9.00 ml |
| | 100.00 ml |

Experiments which have been carried out with the compositions according to the invention have shown that the products are well stabilized and effective after several years storage under normal temperature conditions.

**Claims**

1. Use of thymol and Spiritus vini for preparation of a medicament or cosmetic product for treatment of blemished or scarred skin or skin inflamed with acne (comedones), the amounts of thymol and Spiritus vini used being 0.03-0.75 % and 40-95 ml respectively per 100 ml of the medicament or cosmetic product.

2. Use as claimed in claim 1, wherein the thymol and Spiritus vini are used together with additives commonly used in the cosmetics industry for preparation of the medicament or cosmetic product.

3. Use as claimed in claim 2, wherein the medicament or cosmetic product comprises the thymol and the Spiritus vini, the balance consisting of the additives commonly used in the cosmetics industry.

4. Use as claimed in claim 2 or 3, wherein said additives comprise aqua distillata with or without glycerine.

**Revendications**

4

1. Utilisation de thymol et d'alcool vinique pour la préparation d'un médicament ou d'un produit cosmétique destiné au traitement de la peau abîmée ou portant des cicatrices ou encore de la peau enflammée par l'acné (comédons), les quantités de thymol et d'alcool vinique utilisées étant de 0,03-0,75 g et de 40-95 ml, respectivement, par 100 ml du médicament ou du produit cosmétique.

2. Utilisation selon la revendication 1, dans laquelle on utilise le thymol et l'alcool vinique, conjointement avec des additifs communément utilisés dans l'industrie des cosmétiques pour la préparation du médicament ou du produit cosmétique.

3. Utilisation selon la revendication 2, dans laquelle le médicament ou le produit cosmétique comprend le thymol et l'alcool vinique, le reste étant constitué par les additifs communément utilisés dans l'industrie des cosmétiques.

4. Utilisation selon la revendication 2 ou 3, dans laquelle les additifs comprennent de l'eau distillée avec ou sans glycérine.

**Patentansprüche**

1. Anwendung von Thymol in einer Menge von 0,03 - 0,75% (bezogen auf das Gewicht) und Spiritus vini in einer Menge von 40 - 95 Volumen % zur Herstellung eines Medikaments oder eines kosmetischen Produkts zur Behandlung von entstellter oder vernarbter Haut oder von Akne (Comedones) befallener Haut.

2. Anwendung nach Anspruch 1, wobei Thymol und Spiritus vini zusammen mit Additiven eingesetzt werden, welche in der kosmetischen Industrie zur Herstellung von Medikamenten und kosmetischen Produkten üblicherweise zur Anwendung kommen.

3. Anwendung nach Anspruch 2, wobei das Medikament oder das kosmetische Produkt Thymol und Spiritus vini enthalten, und der Rest aus den Additiven besteht, welche üblicherweise in der kosmetischen Industrie zur Anwendung kommen.

4. Anwendung nach Anspruch 2 oder 3, wobei die besagten Additive Aqua destillata mit oder ohne Glyzerin umfaßt.